(19) Europäisches Patentamt
European Patent Office
Office européen des brevets

(11) Veröffentlichungsnummer: **0 068 291**
**B1**

(12) **EUROPÄISCHE PATENTSCHRIFT**

(45) Veröffentlichungstag der Patentschrift: **06.11.85**

(51) Int. Cl.⁴: **C 12 Q 1/42**, G 01 N 33/548

(21) Anmeldenummer: **82105246.1**

(22) Anmeldetag: **15.06.82**

(54) **Immunochemisches Verfahren und Testkit zur Bestimmung der sauren Prostataphosphatase.**

(30) Priorität: **22.06.81 US 276223**

(43) Veröffentlichungstag der Anmeldung: **05.01.83 Patentblatt 83/1**

(45) Bekanntmachung des Hinweises auf die Patenterteilung: **06.11.85 Patentblatt 85/45**

(84) Benannte Vertragsstaaten: **AT BE CH DE FR GB IT LI NL SE**

(56) Entgegenhaltungen:
**EP - A - 0 032 204**
**US - A - 4 048 298**

**JOURNAL OF BIOLOGICAL CHEMISTRY, Band 243, Nr. 1, 1968 USA H.H. SUSSMAN et al. "Human Alkaline Phosphatase" Seiten 160 to 166**
**CLINICAL CHEMISTRY, Band 26, Nr. 13, 1980 B.K. CHOE et al. "Double-Antibody Immunoenzyme Assay for Human Prostatic Acid Phosphatase" Seiten 1854 bis 1859**
**PROCEEDINGS OF THE SOCIETY FOR EXPERIMENTAL BIOLOGY AND MEDICINE, Band 162, 1979 B.K. CHOE et al. "Immunoenzyme Assay for Human Prostatic Acid Phosphatase" Seiten 396 to 400**

(73) Patentinhaber: **F. HOFFMANN-LA ROCHE & CO. Aktiengesellschaft, CH-4002 Basel (CH)**

(72) Erfinder: **Hager, Hans Jacob, 249 Madisonville Road, Basking Ridge, N.J. (US)**
Erfinder: **Usategui Gomez, Magdalena, 40 Mario Road, Wayne, N.J. (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die sauren Phosphatasen stellen eine heterogene Gruppe von vielen Isoenzymen dar, wobei jedes für einen Gewebetyp spezifisch ist. Als Folge dieser Gewebespezifität ist die Bestimmung eines gewebespezifischen Isoenzyms im Serum eine wertvolle Hilfe in der Identifizierung von Abnormitäten in diesem Gewebe. In einer Zahl von wissenschaftlichen Veröffentlichungen wurde gezeigt, dass die Spiegel des Isoenzyms saure Prostataphosphatase (Prostatic Acid Phosphatase; PAP) im Serum in Begleitung mit der Gegenwart eines Prostatakarzinoms ansteigen (R.M. Townsend, Ann. Clin. and Lab. Sci, Vol., 7, No. 3, pp 254–261, Mai-Juni 1977).

Die Messung oder Bestimmung von PAP-Isoenzymspiegeln im Serum ist jedoch wegen der Gegenwart von andern Isoenzymen der sauren Phosphatase von Gewebe, das nicht aus der Prostata herrührt, kompliziert. Tatsächlich sind normalerweise signifikante Spiegel von Isoenzymen saurer Phosphatase, die nicht von der Prostata stammen, im Serum vorhanden [Li, C. et al., J. Lab. Clin. Med. 82, 446–460 (1973)]. Im Hinblick auf die Vielzahl von Quellen für die Bildung erhöhter Spiegel von Isoenzymen der sauren Phosphatase besteht die Notwendigkeit für eine Bestimmung des Isoenzymes PAP, welche für dieses Isoenzym spezifisch ist.

Frühere Versuche zur Bestimmung der Menge an PAP im Serum wurden auf Substratspezifität und chemische Inhibierung von PAP ausgerichtet. Diese Versuche waren nicht sehr erfolgreich, da sogar das spezifischste Substrat oder der spezifischste Inhibitor mit andern Isoenzymen als PAP reagiert. Diese Versuche gemäss dem Stand der Technik verursachen falsch positive oder negative Testresultate mit der begleitenden hohen Möglichkeit einer unsachgemässen Therapie.

Es wurden auch verschiedene immunochemische Bestimmungsmethoden für PAP beschrieben. Choe, B.K., et al haben in Proc. Soc. Exper. Biol. and Med. 162, 396–400 (1979) eine Methode zur Bestimmung der enzymatischen Aktivität als auch der Radioaktivität eines durch Ammonsulfat gefällten $^{125}$I-markierten PAP Antigen/Antikörperkomplexes beschrieben. Nach dieser Methode wird die PAP durch Messung des durch Ammonsulfat gefällten Antigen/Antikörperkomplexes bestimmt. Nach dieser Methode gibt es jedoch eine hohe Störung durch saure Phosphatasen, welche auf die nicht spezifische Natur der Ammoniumsulfatfällung zurückzuführen sind.

Zur Lösung des Problems der Störung durch andere Isoenzyme in Bestimmungen für individuelle, organspezifische Isoenzyme der menschlichen alkalischen Phosphatase wurde von Sussman et al. in J. Biol. Chem. 243, 160 (1968) und von Choe et al., Clin. Chem. 26, 1854–1859 (1980) ein Doppelantikörperenzymimmunverfahren beschrieben. In beiden dieser Arbeiten wird beschrieben, dass der spezifische Antikörper für das gewünschte Isoenzym mit der Testprobe zur Reaktion gebracht wird worauf in einem nachfolgenden Schritt der Antikörper/Isoenzymkomplex mit einem zweiten löslichen Antikörper (anti-μ-Globulin) zur Reaktion gebracht wird. Nach der Zentrifugation wird der Niederschlag auf Rest-Isoenzymaktivität getestet.

Damit das Sussman- und Choeverfahren präzis verläuft, ist es notwendig, dass der zweite lösliche Antikörper in der Weise mit dem Antikörper/Isoenzymkomplex reagiert, dass das zu bestimmende Isoenzym vollständig gefällt wird. Diese Reaktion ist von Faktoren, wie Temperatur, pH, Ionenstärke, Zeit, Proteinkonzentration und dergleichen abhängig. Da diese Faktoren in einem Bestimmungsverfahren schwierig zu kontrollieren sind, haben das Sussman und Choe Verfahren beschränkte Brauchbarkeit in genauen Bestimmungen der Menge eines spezifischen Isoenzyms.

Die vorliegende Erfindung bezieht sich auf ein immunochemisches Verfahren zur Bestimmung der Menge des PAP-Isoenzyms in einer Probe einer biologischen Flüssigkeit. Erhöhte Spiegel von PAP-Isoenzym in Serum zeigt ein Prostatakarzinom an.

Das immunochemische Verfahren gemäss der vorliegenden Erfindung ist ein Doppelantikörperimmunenzymverfahren, bei dem einer der Antikörper ein unlöslich gemachter Antikörper ist, welcher durch Reaktion dieses Antikörpers mit Amylose gebildet wird. Das Verfahren umfasst die Inkubation der Testprobe mit einem ersten Antikörper, der selektiv immunologisch an das Isoenzym in der Probe bindet und einem unlöslich gemachten zweiten Antikörper, der selektiv immunologisch an den ersten Antikörper bindet. Die resultierenden immunochemischen Reaktionen liefern unlösliche Immunkomplexe, welche einfach zu isolieren sind. Nach der Isolierung der Immunkomplexe werden in diesen die enthaltenden Isoenzymaktivitäten gemessen.

Es wurde gefunden, dass Proteine, wie Immunglobuline (IgG), Enzyme und dergleichen an Amylose, einen festen Träger, gekuppelt werden können und unlöslich gemachte Proteine, wie IgG-Amylose, liefern, welche als Immunabsorbantien verwendet werden können. Es wurde gefunden, dass ein Protein, wie z.B. IgG, welches durch Reaktion mit Amylose unlöslich gemacht wurde, gegenüber Proteinen, die durch andere Festträger unlöslich gemacht wurden, Vorteile zeigt. Im speziellen liegt der Vorteil darin, dass die IgG-Amylose durch eine sehr einfache Reaktion hergestellt werden kann. Amylose hat eine grosse Oberflächenaera für die Bindung des IgG; das erhaltene Reaktionsprodukt, d.h. die IgG-Amylose, kann bei hohen Geschwindigkeiten einfach zentrifugiert werden, bleibt jedoch für die Durchführung einer immunochemischen Bestimmung lang genug in Suspension.

In der US-P 4 048 298 werden Stärke und Dextrin neben zahlreichen anderen Stoffen als Festphasen für einen zweiten Antikörper in einem Doppelantikörperimmunverfahren offenbart, ohne dass diese besonders hervorgehoben werden oder dass besondere Vorteile geltend gemacht werden. Amylose wird nicht erwähnt.

Das Verfahren gemäss vorliegender Erfindung hat wesentliche Vorteile gegenüber Verfahren gemäss dem Stand der Technik, in dem das erfindungsgemässe Verfahren sehr spezifisch, rasch, äusserst präzis und reproduzierbar ist und eine spezifische Isolierung des PAP-Isoenzyms von Seren erlaubt, die nicht-prostatische Isoenzyme enthalten, und so die spezifische Messung der Menge des isolierten PAP-Isoenzyms erlaubt.

Im speziellen ist das Verfahren gemäss der vorliegenden Erfindung von Vorteil, in dem die resultierenden Immunkomplexe rasch aus dem Reaktionsgemisch ausgefällt werden können und für die Messung der Isoenzymaktivität zur Verfügung stehen. Im weitern ist das Reaktionsgemisch, welches den Immunkomplex bildet, unabhängig von Faktoren, wie Temperatur, pH, Ionenstärke, Zeit, Proteinkonzentration und dergleichen. Da diese Faktoren abwesend sind, werden sie beim Verfahren der vorliegenden Erfindung als mögliche Quellen von Fehlern ausgeschaltet, was bedeutet, dass das Verfahren gemäss vorliegender Erfindung eine genauere Bestimmung der Menge des spezifischen Isoenzyms ermöglicht.

Beim erfindungsgemässen Verfahren ist es möglich, als Testprobe eine Probe irgendeiner biologischen Flüssigkeit, wie z.B. Vollblut, Plasma, Serum, Lymphflüssigkeit, Gallenflüssigkeit, Urin, Spinalflüssigkeit, Speichel, Schweiss und dergleichen, sowie auch Stuhlproben von Menschen oder Tieren zu verwenden. Es ist auch möglich, flüssige Präparate von menschlichem oder tierischem Gewebe, wie Skelettmuskel, Herz, Niere, Lunge, Hirn, Knochenmark, Haut und dergleichen zu verwenden, biologische Flüssigkeiten sind jedoch bevorzugt. Die bevorzugte biologische Flüssigkeit als Testprobe gemäss vorliegender Erfindung ist menschliches Serum.

Die vorliegende Erfindung bezieht sich im speziellen auf ein Verfahren zur Bestimmung der Menge des PAP-Isoenzyms in einer Probe einer biologischen Flüssigkeit, welche andere, nicht-prostatische Isoenzyme oder andere proteinartige Substanzen enthalten kann. Das Verfahren gemäss vorliegender Erfindung wird durch Isolieren des spezifischen PAP-Isoenzyms, das von Interesse ist, und durch Messen der enzymatischen Aktivität des isolierten PAP-Isoenzyms durchgeführt.

Die Isolierung des PAP-Isoenzyms wird durch die Verwendung von zwei verschiedenen Antikörpern, einem ersten und einem zweiten Antikörper, welche in einem Reaktionsgemisch mit der Probe der biologischen Flüssigkeit reagieren, und nachfolgende Trennung der Immunkomplexe enthaltend das PAP-Isoenzym aus diesem Reaktionsgemisch durchgeführt.

Gemäss der vorliegenden Erfindung kann der erste Antikörper jeder Antikörper sein, welcher im Reaktionsgemisch löslich ist und welcher selektiv an das PAP-Isoenzym bindet ohne die enzymatische Aktivität des PAP-Isoenzyms signifikant zu inhibieren. Der erste Antikörper kann in herkömmlicher Weise durch Injektion eines gereinigten PAP-Isoenzyms als Antigen in ein Tier und anschliessende Blutentnahme zur Gewinnung der Seren enthaltend den Antikörper hergestellt werden. Zur Herstellung und Reinigung des PAP-Isoenzyms sowie zur Injektion des gereinigten PAP-Isoenzyms als Antigen in Tiere zur Gewinnung von anti-PAP Serum enthaltend den ersten Antikörper kann jede herkömmliche Technik benutzt werden. Das anti-PAP Serum muss nicht gereinigt werden und kann direkt im erfindungsgemässen Verfahren verwendet werden. Das anti-PAP Serum, das man durch Verwendung von gereinigtem PAP-Isoenzym erhält, reagiert nicht mit sauren Phosphatasen anderer Gewebe als Prostata und inhibiert die enzymatische Aktivität des PAP-Isoenzyms, an welches es immunologisch bindet, nicht signifikant. Das bedeutet also, dass der erste Antikörper, der in diesem anti-PAP Serum enthalten ist, spezifisch und nur an PAP-Isoenzym der Testprobe bindet und gleichzeitig die enzymatische Aktivität des PAP-Isoenzyms nicht beeinträchtigt.

Das PAP-Isoenzym, das als Antigen zur Erzeugung des ersten Antikörpers verwendet wird, kann aus jeder geeigneten biologischen Flüssigkeit, wie Prostataflüssigkeit, Blut, Serum oder aus jedem geeigneten biologisch frischen Prostatagewebe gewonnen werden. Die bevorzugte Quelle dieses Isoenzyms sind tierische Prostataflüssigkeit oder Gewebe, besonders bevorzugt ist menschliche Prostataflüssigkeit oder Gewebe.

Um die Gegenwart nicht spezifischer Antikörper herabzusetzen, ist es empfehlenswert, das PAP-Isoenzym zu einem hohen Reinheitsgrad zu reinigen, bevor es zur Erzeugung des ersten Antikörpers verwendet wird. Das PAP-Isoenzym kann nach jeder herkömmlichen Technik gereinigt werden, welche für derartige Zwecke bekannt ist. Die bevorzugte Reinigung umfasst klassische, bekannte Methoden, wie Affinitätschromatographie oder Elektrophorese.

Der Reinheitsgrad des PAP-Isoenzyms, welches zur Erzeugung des ersten Antikörpers verwendet wird, sowie die Spezifität des erhaltenen Antikörpers kann nach herkömmlichen Methoden wie Immundiffusion oder elektrophoretische Techniken bestimmt werden. Die bevorzugte Methode für die Bestimmung der Reinheit des PAP-Isoenzyms ist die Acrylamidgelelektrophorese.

Der erste Antikörper kann in jeder für diesen Zweck bekannten Tierspezies gewonnen werden. Die Tierspezies umfasst insbesondere Vertebraten, wie Schwein, Rindvieh, Hund, Esel, Pferd, Ziege, Kaninchen, Ratte und dergleichen. Unter diesen Tieren sind die Säugetiere, wie Esel, Schaf und Ziege, besonders bevorzugt. Speziell bevorzugt sind Ziegen.

In einem bevorzugten Aspekt der vorliegenden Erfindung wird der erste Antikörper durch Immunisieren von Ziegen, einer ersten Tierspezies, mit gereinigtem PAP-Isoenzym nach herkömmlichen immunologischen Techniken und Gewinnung des Ziegen anti-PAP erhalten. Das gereinigte PAP-Isoenzym wird aus menschlichem Prostatagewebe erhalten.

Der erste Antikörper wird in einer Menge verwendet, die ausreicht, das PAP-Isoenzym der Probe zu binden. Zur Erzielung von guten Resultaten wird der Antikörper der Probe in einer Menge zugesetzt, die oberhalb derjenigen liegt, die gewöhnlich zur Bindung des PAP-Isoenzyms ausreicht. Die Bestimmung der Menge des benötigten ersten Antikörpers für jede Probe kann nach herkömmlichen Methoden erfolgen.

Nach Zugabe des ersten Antikörpers wird die Testprobe mit einem unlöslich gemachten zweiten Antikörper behandelt. Die Zeit für die Zugabe des unlöslich gemachten zweiten Antikörpers nach der Zugabe des ersten Antikörpers ist nicht kritisch, doch ist es bevorzugt, mit der Zugabe des unlöslich gemachten zweiten Antikörpers wenigstens 15 Minuten nach Zugabe des ersten Antikörpers zu warten.

Gemäss der vorliegenden Erfindung wird ein unlöslich gemachter zweiter Antikörper durch Kuppeln des zweiten Antikörpers über eine Aminbrücke an Amylose bereitgestellt. Der zweite Antikörper dieses unlöslich gemachten zweiten Antikörpers kann jeder Antikörper sein, der selektiv an den ersten Antikörper bindet ohne die enzymatische Aktivität des an diesen ersten Antikörper gebundenen Isoenzyms zu inhibieren. Der zweite Antikörper dieses unlöslich gemachten zweiten Antikörpers kann nach jeder herkömmlichen Technik in einer Tierspezies hergestellt werden, die unterschiedlich ist von der, in der der erste Antikörper hergestellt wurde. Der zweite Antikörper wird durch Immunisieren eines Tieres, welches unterschiedlich ist von dem, in dem der erste Antikörper hergestellt wird, mit Gammaglobulin des Blutes der Wirtsspezies, welche zur Herstellung des ersten Antikörpers verwendet wird, hergestellt. Auf diese Weise wird der zweite Antikörper immunoreaktiv für den ersten Antikörper und bildet einen Komplex mit ihm.

Die zur Bildung des unlöslich gemachten zweiten Antikörpers verwendete Amylose kann ein Homopolymer von D-Glukose sein. Die D-Glukopyranose-Einheiten in der Amylose sind im wesentlichen in (1–4) α-glycosidischer Bindung. Das Molekulargewicht der Amylose variiert abhängig von der Amylosequelle und erstreckt sich über einen Bereich von einem durchschnittlichen Molekulargewicht von ungefähr 100 000 bis ungefähr 1 100 000.

Gemäss dem Verfahren der vorliegenden Erfindung kann zur Bildung des Reaktionsproduktes des Antikörpers und der Amylose eine Amylose jeder Quelle und jedes Molekulargewichtes verwendet werden. Falls erwünscht, kann die Amylose in herkömmlicher Weise vor der Reaktion mit dem Antikörper gereinigt werden. Der Grad der Reinheit ist dagegen nicht kritisch, doch ist bevorzugt, dass die Amylose eine Reinheit zwischen 50 und 100% hat.

Das Verfahren zum Binden des zweiten Antikörpers oder eines anderen Proteins via eine Aminbrücke an die Amylose zur Bildung des unlöslich gemachten zweiten Antikörpers kann jede herkömmliche Technik für die Kupplung von Polysacchariden an Proteine sein.

Nach einem bevorzugten Aspekt der vorliegenden Erfindung, wird der zweite Antikörper oder ein anderes Protein unter Verwendung einer konventionellen Schiffbase-Reaktion kovalent an aktivierte Amylose gekuppelt (Guthrie, R.D. Adv. Carbohydrate Chem. 16:101, 1961).

Gemäss dem bevorzugten Verfahren wird Amylose, welche eine Mehrzahl von D-Glucopyranose-Einheiten enthält, mit Hilfe eines Oxidationsmittels, wie Natriumperiodat ($NaIO_4$) aktiviert, welches die α-1,4-gebundenen Glucoseeinheiten oxidiert und Amylosedialdehyd liefert. Der erhaltene Amylosedialdehyd reagiert dann mit der primären Aminogruppe des zugegebenen zweiten Antikörpers oder des Proteins und formt eine Schiffbase oder ein Aldimin. Die Schiffbase wird mit einem Reduktionsmittel behandelt und liefert eine primäre Aminobrücke zwischen dem Protein und der Amylose. Unter den herkömmlichen, für diesen Zweck geeigneten Reduktionsmitteln wird gewöhnlich ein Reduktionsmittel wie Natriumborhydrid ($NaBH_4$) verwendet. Das Verfahren zur Herstellung des zweiten Antikörper/Amyloseproduktes wird im folgenden Reaktionsschema illustriert. In diesem Schema bedeutet n eine Vielzahl von D-Glucosepyranose-Einheiten.

Reaktionsschema für die Amylose/Protein Kupplung

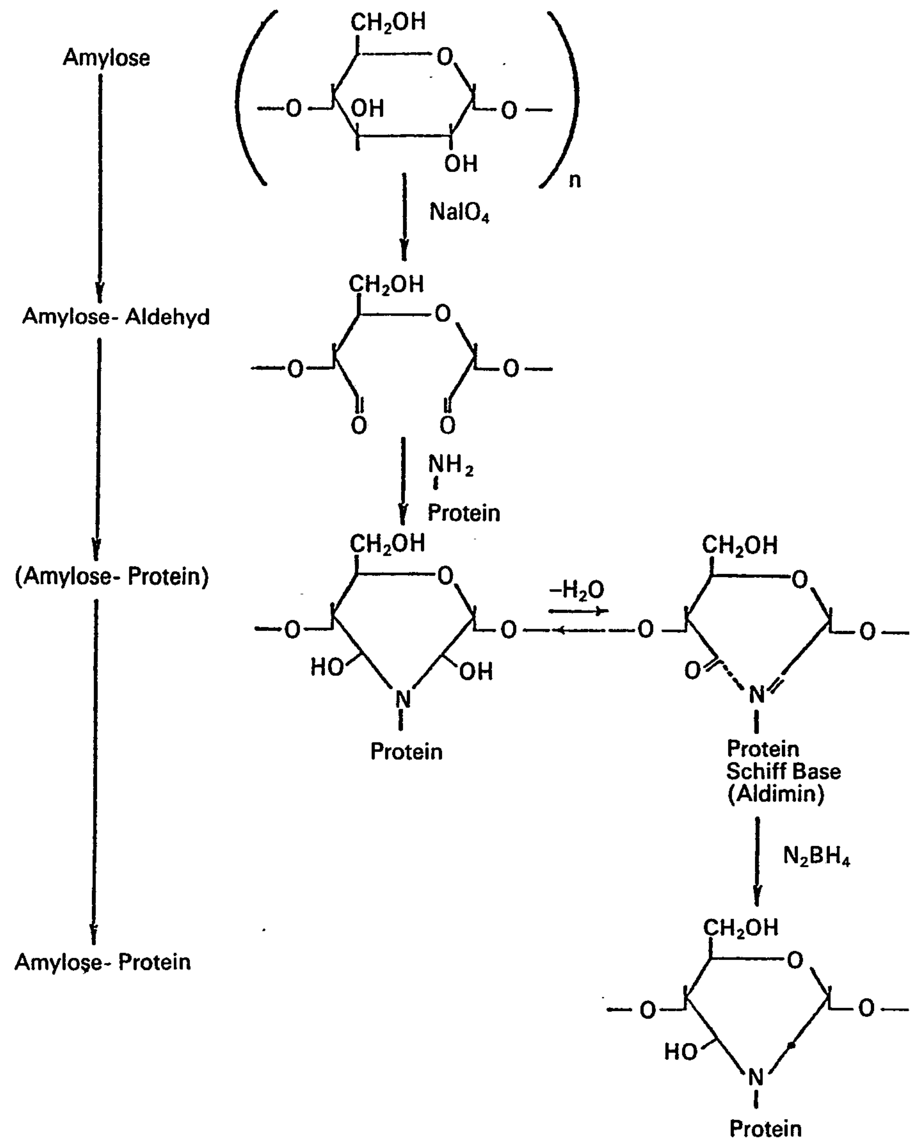


Zur Bildung des unlöslich gemachten Antikörpers wird der Antikörper in der vorerwähnten Weise mit Amylose zur Reaktion gebracht um den Antikörper via eine Aminbrücke an die Amylose zu kuppeln. Bei der Herstellung des unlöslich gemachten Antikörpers sind die Mengen an Amylose und Antikörper nicht kritisch. Es werden jedoch gewöhnlich ungefähr 1 bis ungefähr 200 Gewichtsteile Amylose pro Gewichtsteil Antikörper verwendet. Bevorzugt ist die Verwendung von ungefähr 8 bis ungefähr 20 Gewichtsteilen Amylose pro Gewichtsteil Antikörper.

Gemäss der vorliegenden Erfindung werden die durch die Zugabe des zweiten Antikörpers gebildeten Immunkomplexe durch herkömmliche Techniken aus der Probenlösung oder -mischung getrennt, und im erhaltenen Niederschlag wird die enzymatische Wirkung der PAP gemessen. Gemäss der vorliegenden Erfindung wird die enzymatische Aktivität also im Niederschlag gemessen.

Zu den herkömmlichen Methoden für die Abtrennung des Niederschlages aus der Probenlösung gehören die Filtration und chromatographische Methoden. Die bevorzugteste Methode zur Trennung des Niederschlages von der Probenlösung ist die Zentrifugation mit anschliessender Dekantierung des Überstandes und Messung des Niederschlages. Dies erlaubt die Messung der Menge von PAP in der Probe.

Die Messung oder Bestimmung der Aktivität des PAP-Isoenzyms, welches aus der Testprobe isoliert wurde, kann nach jeder herkömmlichen Methode erfolgen. Diese Methoden umfassen colorimetrischen Methoden, wie sie z.B. in «Methoden der enzymatischen Analyse» herausgegeben von H.U. Bergmeyer, dritte Ausgabe 1974, Vol. 1, Seite 145 ff. beschrieben sind sowie auch fluorimetrische Methoden.

Die Erfindung betrifft auch ein diagnostisches Testkitsystem zur quantitativen Bestimmung der Gegenwart des PAP-Isoenzyms in einer biologischen Flüssigkeit. Die Bestandteile des Testkitsystems sind: (a) ein erster Behälter enthaltend einen Antikörper, der fähig ist, selektiv an das PAP-Isoenzym zu binden, (b) einen zweiten Behälter enthaltend einen zweiten Antikörper der durch Kuppeln an Amylose über eine Aminbindung unlöslich gemacht wurde und der in der Lage ist, selektiv immunologisch mit dem ersten Antikörper enthalten im ersten Behälter zu reagieren und (c) ein Substratreagens, mit welchem die enzymatische Aktivität des PAP-Isoenzyms gemessen werden kann.

Wenn alle Komponenten des diagnostischen Testkitsystems gemäss vorliegender Erfindung verwendet werden, ist der Fachmann in der Lage, die enzymatische Aktivität des PAP-Isoenzyms in einer Probe einer biologischen Flüssigkeit zu bestimmen, welche nicht-prostatische Isoenzyme enthält. Das Testkit ist geeignet für Kliniken, Spitäler Laboratorien und individuelle Ärzte, welche die Menge von PAP-Isoenzym in einer Probe einer biologischen Flüssigkeit bestimmen müssen.

Die nachfolgenden Beispiele illustrieren die Erfindung weiterhin, sollen aber den Umfang und den Geist der Erfindung nicht beschränken.

Beispiel 1
Reinigung von PAP

Teil A

100 g gefrorenes humanes Prostatagewebe werden bei 4 °C in eine Homogenisierungslösung enthaltend 0,05 M Äthylendiamintetraessigsäure (EDTA) und 0,01% (Gewicht) Tween 80 gegeben. Man lässt das gefrorene Gewebe in der Homogenisierungslösung während ungefähr einer halben Stunde auftauen. Nach dem Auftauen wird das Gewebe in einem Mischer homogenisiert, bis in der resultierenden Lösung keine Klumpen mehr vorhanden sind. Während der Homogenisierung, die nicht länger als 20 Sekunden durchgeführt wird, befindet sich die Mischung in einem Eisbad. Das resultierende homogenisierte Material wird darauf über Nacht bei 4 °C gerührt. Das Material wird dann während einer Stunde bei 4 °C und 16,300 Xg zentrifugiert. Der erhaltene Überstand von ungefähr 240 ml wird dreimal gegen 4 Liter 0,005 M Natriumphosphatpuffer vom pH 6,3 enthaltend 0,9% Natriumchlorid dialysiert. Durch die Dialyse erhöht sich das ursprüngliche Volumen von ungefähr 240 ml auf ungefähr 260 ml.

Das erhaltene dialysierte Material wird dann in eine 1000 ml Lyophislisierungsflasche gegeben, welche für Lyophilisation durch Schnellfrierung über Trockeneis und Aceton geeignet ist, gegeben und dann über Nacht lyophilisiert. Das erhaltene lyophilisierte Material wird dann mit 20,0 ml 0,005 M Natriumphosphatpuffer vom pH 6,3 enthaltend 0,9% Natriumchlorid rekonstituiert, wobei man das rekonstituierte Material enthält, welches für den nachfolgenden Teil B verwendet wird.

Teil B

Eine LKB-Kolonne wird mit 100 ml eines im vorerwähnten Puffer vorgequollenen Con-A Sepharose 4B-Gel gepackt, worauf sich das Gel ohne Durchflussrate in der Kolonne setzen kann. Die erhaltene gepackte Kolonne wird dann mit 400 ml des oben erwähnten Puffers gewaschen.

Das rekonstituierte Material vom obigen Teil A wird auf die so präparierte LKB-Kolonne gegeben. Darauf lässt man das Material sich während 3 Stunden auf der Kolonne absetzen. Darauf werden 800 ml des vorerwähnten Puffers mit einer Durchflussrate von 40 ml pro Stunde durch die Kolonne gelassen. Darauf werden 500 ml eines 0,0 bis 0,3 M Mannose-Gradienten, welcher separat in 0,005 M Natriumphosphatpuffer vom pH 6,3 mit 0,9% Natriumchlorid und Filtrieren durch Aktivkohle erhalten wurde, mit einer Durchflussrate von 40 ml pro Stunde durch die Kolonne gelassen, wobei 10 ml Fraktionen gesammelt werden (total 50 Fraktionen). Darauf werden 500 ml 1,0 M Mannose durch die Kolonne geleitet, und es werden in der gleichen Weise insgesamt 50 Fraktionen gesammelt. Alle Fraktionen werden auf Protein und PAP-Aktivität getestet. Fraktionen enthaltend eine PAP-Aktivität mit einer spezifischen Aktivität ⩾ 2000 Sigma Einheiten/ml Protein werden vereinigt, gegen 0,05 M Natriumphosphatpuffer vom pH 6,3 mit 0,9% Natriumchlorid dialysiert und unter Verwendung eines Amiconfiltersystems zu einem 4,0 ml konzentriertem Dialysat für den nachfolgenden Teil C konzentriert.

Teil C

Man stelle ein 7,5%-iges Acrylamidgel unter Verwendung eines Büchlersystems her. Die nchfolgenden Puffer wurden ebenfalls für das Büchlersystem hergestellt: 0,035 M β-Alanin in 0,014 M Essigsäure für den oberen Puffer des Systems und 0,14 M β-Alanin in 0,56 M Essigsäure für den unteren und Elutionspuffer des Systems.

Zur Herstellung eines Gemisches werden 1 ml 40%-ige Sucrose und 3 Tropfen Methylgrün-Farbe zu den 4 ml des konzentrierten Dialysates von Teil B gegeben. Das Gemisch wird dann auf das 7,5%-ige Acrylamidgel des Büchlersystems gegeben und die Elektrophorese wird gestartet (1000 Volt, 200 Miliampère während ungefähr 16 Stunden). Das Gemisch wird unter Verwendung des unteren/Elutionspuffer eluiert, wobei 10 ml Fraktionen gesammelt werden. Die erhaltenen Fraktionen werden auf PAP und Proteinaktivität untersucht. Fraktionen enthaltend PAP-Aktivität werden vereinigt und über Nacht zweimal gegen 0,01 M Citratpuffer vom pH 6,0 dialysiert, wobei man hochgereinigtes PAP für das Immunisierungsverfahren gemäss nachfolgendem Beispiel 2 erhält. Das hochgereinigte PAP kann bei −20 °C gelagert werden.

Beispiel 2
Immunisierung von Ziegen mit gereinigtem PAP zur Erzeugung des ersten Antikörpers:

Zur Herstellung des Immunogens zur Immunisierung der Ziegen wird das gemäss Beispiel 1 erhaltene hochgereinigte PAP in 0,01 M Citratpuffer vom pH 6,0 mit einem gleichen Volumen von

Freund's komplettem Adjuvans (eine Mineralölsuspension enthaltend abgetötete M-Tuberkulosisbazillen) gemischt, wobei man eine Mischung enthaltend 0,5 mg PAP/ml erhält. Zur Bildung der Wasser/Ölemulsion, welche das Immunogen darstellt, wird das erhaltene Gemisch homogenisiert. Ziegen werden während drei aufeinanderfolgenden Wochen mit einer subkutanen Injektion von 2 ml Immunogen in 2 Stellen der Achselgegend immunisiert, wobei jede Stelle 1 ml des Immunogens erhält. Die Zweitinjektionen mit inkompletten Freund's Adjuvans werden in zwei Wochenintervallen nach den ersten Injektionen gegeben, worauf man weitere Zweitinjektionen mit inkompletem Freund's Adjuvans während mehreren Monaten monatlich durchführt. Den Ziegen wird jeweils eine bis zwei Wochen nach der Immunisierung Blut entnommen um Antiserum zu erhalten.

Beispiel 3

Herstellung der Globulinfraktion von Esel-anti-Ziegen IgG-Serum

Die Globulinfraktion von Esel-anti-Ziegen IgG-Serum, welche für die Kupplung an die Amylose gebraucht wird, wird unter Verwendung der folgenden Ingredienten und Verfahren hergestellt:

| Punkt | Bestandteil | Menge |
|---|---|---|
| a) | Kommerziell erhältliches Esel-anti-Ziegen IgG-Serum | 500 ml |
| b) | gesättigte Ammoniumsulfatlösung; hergestellt durch Zufügen von 500 g festem Ammoniumsulfat (Granulat) zu 500 ml entionisiertem Wasser und heftigem Rühren während einer Stunde bei Raumtemperatur. Die Lösung wird bei 4 °C gelagert, wobei sich die Kristalle absetzen. | 550 ml |
| c) | 0,01 M Natriumphosphatpuffer vom pH 7,4 ungefähr | 14 Liter |
| d) | 0,01 M Natriumcarbonatbicarbonatpuffer vom pH 9,5. Hergestellt durch Lösen von 1,1 g Natriumcarbonat und 2,5 g Natriumbicarbonat in ungefähr 3,5 Liter entionisiertem Wasser, Rühren während 5–10 Minuten und Auffüllen auf ein Volumen von 4 Liter mit entionisiertem Wasser. | 5 Liter |

500 ml Esel-anti-Ziegen IgG-Serum werden in ein geeignetes Gefäss mit Magnetrührer gegeben. Um den Gefässinhalt zu 37,5% zu sättigen, werden unter schwachem Rühren 300 ml gesättigte Ammoniumsulfatlösung (Punkt b) zugegeben. Nach einstündigem Rühren bei 4 °C wird der ausgefällte Antikörper durch Zentrifugation bei 1200 Xg während 30 Minuten bei 4 °C als Kügelchen gesammelt. Das Kügelchen wird in ungefähr 350 ml Phosphatpuffer (Punkt c) gelöst, worauf man mit demselben Puffer wieder das Originalvolumen von 500 ml herstellt. Um die Mischung auf eine 33%-ige Sättigung zu bringen, werden unter leichtem Rühren bei 4 °C 250 ml gesättigte Ammoniumsulfatlösung (Punkt b) zugegeben. Das Gemisch wird während einer Stunde bei 4 °C gerührt. Der ausgefallene Antikörper wird nach 30 minütiger Zentrifugation bei 1200 Xg und 4 °C als Kügelchen erhalten. Das Kügelchen wird in ungefähr 200 ml des Phosphatpuffers gelöst, dann in einen Dialyseschlauch gegeben und gegen den Phosphatpuffer bei 4 °C dialysiert. Der Puffer wird nach einem Minimum von 4 Stunden Dialyse dreimal gewechselt (4 Liter pro Wechsel). Das erhaltene Dialysat wird erneut dialysiert und zwar gegen 4 Liter Carbonatbicarbonatpuffer (Punkt d). Die Proteinkonzentration dieses Dialysats wird nach der Lowry-Methode bestimmt, und man verdünnt auf 12 mg/ml in einem Endvolumen von 800 ml, wobei man die Globulinfraktion von Esel-anti-Ziegen IgG-Serum erhält, welche zur Kupplung an die aktivierte Amylose gemäss Beispiel 4 geeignet ist.

Beispiel 4

Aktivierung von Amylose

Um die Globulinfraktion von Esel-anti-Ziegen IgG-Serum gemäss Beispiel 3 an Amylose zu kuppeln, wird die Amylose nach den folgenden Stufen 1–16 zuerst aktiviert:

1. Man gibt langsam 20 g Amylose in 4 l Literzentrifugenbehälter enthaltend 500 ml entionisiertes Wasser. Während und nach der Zugabe wird mit einem Magnetrührer gerührt. Die Behälter werden bis zum vollen Volumen mit entionisiertem Wasser gefüllt.
2. Unter einstündigem Rühren bei Raumtemperatur lässt man die Amylose in jedem Behälter quellen.
3. Die gequollene Amylose wird in jedem Behälter während 5 Minuten bei 2500 Xg zentrifugiert.
4. Der Überstand jedes Behälters wird abdekantiert und das Kügelchen für Stufe 5 aufbewahrt.
5. In jedem Behälter wird das Kügelchen in einem Liter deonisiertem Wasser resuspendiert.
6. Die Stufen 3, 4 und 5 werden zweimal wiederholt, was insgesamt 4 Waschvorgänge ergibt.
7. Die Kügelchen werden in Wasser resuspendiert und über Nacht bei Raumtemperatur im Wasser belassen. Am nächsten Tage werden die Behälter zentrifugiert, worauf nach Abdekantieren die Kügelchen für den späteren Schritt 9 aufbewahrt werden.
8. Man löst 25,68 g Natriummetaperiodat in 700 ml deonisiertem Wasser und füllt auf ein Volumen von 800 ml auf.
9. Die Kügelchen gemäss Stufe 7 werden in jedem der vier Behälter mit 200 ml des 0,15 M Natriummetaperiodat gemäss Stufe 8 resuspendiert.

10. Die resuspendierten Kügelchen gemäss Stufe 9 werden während zwei Stunden bei Raumtemperatur leicht gerührt.

11. 49,66 g Äthylenglykol (flüssig) werden mit 300 ml entionisiertem Wasser gemischt. Mit deionisiertem Wasser füllt man auf ein Volumen von 400 ml auf; vor der Zufügung von Äthylenglykol erfolgt keine Zentrifugation.

12. Am Ende des 2-stündigen Rührens gemäss Stufe 10 werden in jedem der vier Behälter 100 ml der 2 M Äthylenglycollösung gemäss Stufe 11 gegeben, worauf man während 30 Minuten bei Raumtemperatur leicht rührt.

13. Die Behälter werden während 5 Minuten bei 2500 Xg zentrifugiert. Nach dem Dekantieren werden die Kügelchen separat voneinander zurückbehalten.

14. Die Kügelchen gemäss Stufe 13 werden separat in bis zu 1 Liter (Gefässinhalt) Carbonatbicarbonatpuffer (Punkt d von Beispiel 3) resuspendiert, worauf man während 15 bis 30 Minuten rührt.

15. Die Stufen 13 und 14 werden zweimal wiederholt.

16. Durch Dekantieren erhält man Kügelchen, welche aktivierte Amylose enthalten, welche bereit ist für das Kuppeln an die Globulinfraktion von Esel-anti-Ziegen IgG-Serum hergestellt gemäss Beispiel 3.

Beispiel 5

Kupplung der Globulinfraktion von Esel-anti-Ziegen IgG-Serum an aktivierte Amylose

Das Kuppeln der Globulinfraktion des Esel-anti-Ziegen IgG-Serums (hergestellt nach dem Verfahren von Beispiel 3) an aktivierte Amylose (hergestellt nach dem Verfahren gemäss Beispiel 4) wird unter Verwendung der folgenden Bestandteile und Verfahren bewerkstelligt.

| Punkt | Bestandteil | Quantität |
|---|---|---|
| 1 | Aktivierte Amylose (Beispiel 4) | 400 ml gequollenes Gel |
| 2 | Esel-anti-Ziegen IgG (Beispiel 3) | 800 ml |
| 3 | Phosphatgepufferte Kochsalzlösung (PBS) pH 7,4 | ungefähr 27 Liter |
| 4 | Natriumborhydrid | 0,8 g |
| 5 | Rinderserumalbumin | 4,8 g |
| 6 | Natriumazid | 1,6 g |

Verfahren

1. Zu jedem der 4 Gefässe enthaltend je 1 Kügelchen von aktivierter Amylose (20 g Trockengewicht, wie in Beispiel 4 angegeben zu 100 ml gequollen) werden 200 ml Esel-anti-Ziegen IgG (hergestellt gemäss Beispiel 3; 12 mg/ml) zur Kupplung an die Amylose an das IgG gegeben.

2. Die Kupplung erfolgt bei Raumtemperatur während 36–38 Stunden unter Rühren.

3. Die Behälter werden dann während 5 Minuten bei 2500 Xg zentrifugiert.

4. Das Protein der Überstände jedes Gefässes wird auf O.D. bei 280 geprüft.

5. Zu 800 ml phosphatgepufferter Kochsalzlösung werden 0,8 g Natriumborhydrid gegeben.

6. Zu jedem der vier zentrifugierten Gefässe gemäss Stufe 3 enthaltend Amylose-Protein werden 200 ml Natriumbohrydridlösung gegeben. Die resultierenden Suspensionen werden während einer Stunde bei Raumtemperatur leicht gerührt.

7. Nach dem Rühren der Suspensionen gemäss Stufe 6 wird das resultierende reduzierte Amylose-Proteingel jedes Gefässes wie in Stufe 3 zentrifugiert, worauf der Überstand verworfen wird.

8. Das Gel in jedem Gefäss wird mit phosphatgepufferter Kochsalzlösung resuspendiert und dann bis zu einem Liter mit Phosphatpuffer aufgefüllt. Das resuspendierte Gel wird während 10 bis 20 Minuten bei Raumtemperatur gerührt.

9. Nach dem Rühren wird das Gel wie in Stufe 3 zentrifugiert. Der Überstand wird verworfen, und die Stufen 3 und 9 werden zweimal wiederholt.

10. Die 4 resultierenden Amylose-Proteinkügelchen werden in je 200 ml PBS resuspendiert und dann mit Waschen in ein 2-Litergefäss gebracht, wobei man eine Amylose-Proteinsuspension erhält.

11. 4,8 g Rinderserumalbumin und 1,6 g Natriumazid werden unter Rühren zu 100 ml PBS gegeben. Diese Lösung wird der Amylose-Proteinsuspension zugegeben.

12. Die Amylose-Proteinsuspension wird dann zu einer 5%-igen wässrigen Suspension gebracht, was durch Zugabe von PBS zu einem Endvolumen von 1600 ml erreicht wird.

Beispiel 6

Die Bestimmung der PAP-Aktivität in einer Probe einer biologischen Flüssigkeit wird wie folgt durchgeführt:

Teil A

200 µl Testserumprobe oder Kontrolle werden in ein Teströhrchen gebracht; für den Reagenzienleerwert werden 200 µl destilliertes Wasser zu einem anderen Teströhrchen gegeben. 20 µl des gemäss Beispiel 2 erhaltenen Ziegen-anti-PAP Serum wird zu jedem Röhrchen gegeben. Nachdem das letzte Röhrchen mit Anti-PAP-Serum versetzt worden ist, werden die erhaltenen Mischungen während 15 Minuten bei Raumtemperatur gemischt und inkubiert. Nachher werden jedem Röhrchen 200 µl einer gut gerührten Amylose-Proteinsuspension gemäss Beispiel 5 zugegeben. Nachdem das letzte der zu untersuchenden Röhrchen mit der Amylose-Proteinsuspension versetzt worden ist werden die Röhrchen gemischt und während 15 Minuten bei Raumtemperatur inkubiert. Darauf werden 3,0 ml einer 0,9%-igen Salzlösung (9,0 g Natriumchlorid pro 1000 ml destilliertes Wasser) zu jedem Röhrchen gegeben, worauf man mischt. Jedes Röhrchen wird dann während 10 Minuten bei 1500 Xg zentrifugiert, worauf der Überstand durch Dekantieren entfernt wird. Man erhält so Röhrchen enthaltend ein Festphasenkügelchen für die Verwendung in Teil B.

Teil B

Die colorimetrische PAP-Substratlösung wird hergestellt durch Rekonstituieren eines Röhrchens Worthington Natriumthymolphthaleinmonophosphat mit 41,0 ml destilliertem Wasser. Die Flasche wird leicht geschüttelt um das Substrat vollständig zu lösen. Diese Substratlösung kann bis zu 60 Tagen bei 2–8 °C oder 2 Tage bei 18–26 °C gelagert werden. Die Substratlösung wird dann auf Raumtemperatur äquilibriert. Die Röhrchen enthaltend das Festphasenkügelchen von Teil A werden in ein 37 °C Wasserbad gestellt und während 5 Minuten äquilibriert. Hiernach werden 0,5 ml der colorimetrischen PAP-Substratlösung zu jedem Röhrchen enthaltend dieses Kügelchen gegeben. Jedes Röhrchen wird dann gemischt und während 30 Minuten bei 37 °C inkubiert (nach Zugabe der Substratlösung zum ersten Teströhrchen). Am Ende der 30-Minutenperiode werden zu jedem Röhrchen 1,5 ml Alkali Worthington-Lösung gegeben, worauf man mischt und dann während 10 Minuten bei 1500 Xg zentrifugiert. Die Absorption des resultierenden Überstandes jedes Röhrchen wird bei 590 nm in einem Spektrometer abgelesen.

Teil C

Die colorimetrische PAP-Substratlösung, die in Teil B verwendet wird, erhält man wie folgt:

Um die Konzentrationen an PAP in den Proben zu bestimmen wird unter Verwendung konventioneller Worthington Thymolphthaleinverdünnungen eine Standardkurve erstellt. Nach Substraktion der Absorption des Reagenzienleerwerts von der Absorption der Serumproben und der Kontrollen verwendet man die korrigierten Absorptionen um die Konzentrationen an PAP von der Standardkurve abzulesen.

**Patentansprüche für die Vertragsstaaten BE, CH, DE, FR, GB, IT, LI, NL, SE**

1. Verfahren zur Bestimmung der Menge des Isoenzyms saure Prostataphosphatase in einer Probe einer biologischen Flüssigkeit, dadurch gekennzeichnet, dass man

a) die Probe inkubiert mit

(i) einem löslichen ersten Antikörper, welcher selektiv immunologisch an das Isoenzym in der Probe bindet ohne die enzymatische Aktivität dieses Isoenzyms zu inhibieren, und

(ii) einem unlöslich gemachten Antikörper gebildet durch Kuppeln dieses zweiten Antikörpers an Amylose, wobei der zweite Antikörper selektiv immunologisch an den ersten Antikörper bindet ohne die enzymatische Aktivität des Isoenzyms zu inhibieren, um einen Niederschlag einer Mischung von Immunkomplexen zu erhalten, welche Isoenzym-ersten Antikörper-zweiten Antikörper und ersten Antikörper-zweiten Antikörper enthalten, und dass man

b) die Niederschläge enthaltend die Immunkomplexe isoliert und

c) die Isoenzymaktivität im Niederschlag misst.

2. Verfahren nach Anspruch 1, dadurch gekennzeichnet, dass die Probe der biologischen Flüssigkeit Blutserum ist.

3. Verfahren nach Anspruch 1 oder 2, dadurch gekennzeichnet, dass der erste Antikörper Ziegen-anti-saure Prostataphosphatase und der zweite Antikörper Esel-anti-Ziegen IgG ist.

4. Verfahren nach einem der Ansprüche 1–3, dadurch gekennzeichnet, dass die Isoenzymaktivität durch spektrophotometrische Messung bestimmt wird.

5. Verfahren nach Anspruch 4, dadurch gekennzeichnet, dass die spektrophotometrische Messung colorimetrische Reagentien umfasst.

6. Verfahren nach Anspruch 5, dadurch gekennzeichnet, dass die colorimetrischen Reagenzien Thymolphthaleinmonophosphat oder ein basisches Additionssalz davon umfassen.

7. Diagnostisches Testkitsystem zur Bestimmung der Menge des Isoenzyms saure Prostataphosphatase in einer Probe einer biologischen Flüssigkeit, dadurch gekennzeichnet, dass das Testkitsystem die folgenden Bestandteile enthält:

a) einen ersten Behälter enthaltend einen Antikörper, welcher in der Lage ist, selektiv immunologisch an das Isoenzym zu binden,

b) einen zweiten Behälter enthaltend einen unlöslich gemachten Antikörper gebildet durch Kuppeln dieses zweiten Antikörpers an Amylose, wobei der zweite Antikörper in der Lage ist, selektiv immunologisch mit dem Antikörper enthalten im ersten Behälter zu reagieren und

c) ein Reagens, mit dem die enzymatische Aktivität des Isoenzyms bestimmt werden kann.

8. Eine Zusammensetzung bestehend aus einem Protein, welches über eine Aminbrücke an Amylose gekuppelt ist.

9. Zusammensetzung nach Anspruch 8, dadurch gekennzeichnet, dass das Protein ein Immunglobulin ist.

10. Verfahren zur Herstellung einer Zusammensetzung nach Anspruch 8 oder 9, dadurch gekennzeichnet, dass man das Protein über eine Aminbrücke an die Amylose kuppelt.

**Patentansprüche für den Vertragsstaat AT**

1. Verfahren zur Bestimmung der Menge des Isoenzyms saure Prostataphosphatase in einer Probe einer biologischen Flüssigkeit, dadurch gekennzeichnet, dass man

a) die Probe inkubiert mit

(i) einem löslichen ersten Antikörper, welcher selektiv immunologisch an das Isoenzym in der Probe bindet ohne die enzymatische Aktivität dieses Isoenzyms zu inhibieren, und

(ii) einem unlöslich gemachten Antikörper gebildet durch Kuppeln dieses zweiten Antikörpers an Amylose, wobei der zweite Antikörper selektiv immunologisch an den ersten Antikörper bindet ohne die enzymatische Aktivität des Isoenzyms zu inhibieren, um einen Niederschlag einer Mischung von Immunkomplexen zu erhalten, welche Isoenzym-ersten Antikörper-zweiten Antikörper und ersten Antikörper-zweiten Antikörper enthalten, und dass man

b) die Niederschläge enthaltend die Immunkomplexe isoliert und

c) die Isoenzymaktivität im Niederschlag misst.

2. Verfahren nach Anspruch 1, dadurch gekennzeichnet, dass die Probe der biologischen Flüssigkeit Blutserum ist.

3. Verfahren nach Anspruch 1 oder 2, dadurch gekennzeichnet, dass der erste Antikörper Ziegen-anti-saure Prostataphosphatase und der zweite Antikörper Esel-anti-Ziegen IgG ist.

4. Verfahren nach einem der Ansprüche 1–3, dadurch gekennzeichnet, dass die Isoenzymaktivität durch spektrophotometrische Messung bestimmt wird.

5. Verfahren nach Anspruch 4, dadurch gekennzeichnet, dass die spektrophotometrische Messung colorimetrische Reagentien umfasst.

6. Verfahren nach Anspruch 5 dadurch gekennzeichnet, dass die colorimetrischen Reagenzien Thymolphthaleinmonophosphat oder ein basisches Additionssalz davon umfassen.

7. Diagnostisches Testkitsystem zur Bestimmung der Menge des Isoenzyms saure Prostataphosphatase in einer Probe einer biologischen Flüssigkeit, dadurch gekennzeichnet, dass das Testkitsystem die folgenden Bestandteile enthält:

a) einen ersten Behälter enthaltend einen Antikörper, welcher in der Lage ist, selektiv immunologisch an das Isoenzym zu binden,

b) einen zweiten Behälter enthaltend einen unlöslich gemachten Antikörper gebildet durch Kuppeln dieses zweiten Antikörpers an Amylose, wobei der zweite Antikörper in der Lage ist, selektiv immunologisch mit dem Antikörper enthalten im ersten Behälter zu reagieren und

c) ein Reagens, mit dem die enzymatische Aktivität des Isoenzyms bestimmt werden kann.

8. Verfahren zur Herstellung einer Zusammensetzung aus Protein und Amylose, dadurch gekennzeichnet, dass man das Protein über eine Aminbrücke an die Amylose kuppelt.

9. Verfahren nach Anspruch 8, dadurch gekennzeichnet, dass das Protein ein Immunglobulin ist.

**Revendications pour les états contractants BE, CH, DE, FR, GB, IT, LI, NL, SE**

1. Procédé de détermination de la quantité de l'isoenzyme phosphatase prostatique acide dans un échantillon de liquide biologique, caractérisé en ce que

a) on fait incuber l'échantillon avec

(i) un premier anticorps soluble, qui se lie sélectivement de façon immunologique sur l'isoenzyme dans l'échantill on sans inhiber l'activité enzymatique de cet échantillon, et

(ii) un anticorps rendu insoluble formé par couplage de ce second anticorps à l'amylose, le second anticorps se liant sélectivement de façon immunologique sur le premier anticorps sans inhiber l'activité enzymatique de l'isoenzyme, pour obtenir un précipité de complexes immuns, qui contiennent l'isoenzyme-premier anticorps-second anticorps et le premier anticorps-second anticorps, et en ce que

b) on isole les précipités contenant les complexes immuns et

c) on mesure l'activité isoenzymatique dans le précipité.

2. Procédé selon la revendication 1, caractérisé en ce que l'échantillon de liquide biologique est du sérum sanguin.

3. Procédé selon les revendications 1 et 2, caractérisé en ce que le premier anticorps et de l'anti-phosphatase prostatique acide de chèvre et en ce que le second anticorps est de l'IgG anti-chèvre d'âne.

4. Procédé selon l'une des revendications 1–3, caractérisé en ce qu'on détermine l'activité isoenzymatique par mesure spectrophotométrique.

5. Procédé selon la revendication 4, caractérisé en ce que la mesure spectrophotométrique comprend des réactifs colorimétriques.

6. Procédé selon la revendication 5, caractérisé en ce que les réactifs colorimétriques comprennent le monophosphate de thymolphthaléine ou un de ses sels d'addition basiques.

7. Système de trousse expérimentale diagnostique pour la détermination de la quantité de l'isoenzyme phosphatase prostatique acide dans un échantillon de liquide biologique, caractérisé en ce que le système de trousse expérimentale contient les composants suivants:

a) un premier récipient contennt un anticorps, qui est en situation de se lier sélectivement de façon immunologique sur l'isoenzyme,

b) un second récipient contenant un anticorps rendu insoluble formé par couplage de ce second anticorps sur l'amylose, où le second anticorps est en situation de réagir sélectivement de facon immunologique avec l'anticorps obtenu dans le premier récipient et

c) un réactif avec lequel on peut déterminer l'activité enzymatique de l'isoenzyme.

8. Composition constituée d'une proéine qui est couplée à l'amylose par l'intermédiaire d'un pont amine.

9. Composition selon la revendication 8, caractérisée en ce que la protéine est une immunoglobuline.

10. Procédé de préparation d'une composition selon les revendications 8 et 9, caractérisé en ce qu'on couple la protéine à l'amylose par l'intermédiaire d'un pont amine.

**Revendications pour l'état contractant AT**

1. Procédé de détermination de la quantité de l'isoenzyme phosphatase prostatique acide dans un échantillon de liquide biologique, caractérisé en ce que

a) on fait incuber l'échantillon avec

(i) un premier anticorps soluble, qui se lie sélectivement de façon immunologique sur l'isoenzyme dans l'échantillon sans inhiber l'activité enzymatique de cet échantillon, et

(ii) un anticorps rendu insoluble formé par couplage de se second anticorps à l'amylose, le second anticorps se liant sélectivement de façon immunologique sur le premier anticorps sans inhiber l'activité enzymatique de l'isoenzyme, pour

obtenir un précipité de complexes immuns, qui contiennent l'isoenzyme-premier anticorps-second anticorps et le premier anticorps-second anticorps, et en ce que

b) on isole les précipités contenant les complexes immuns et

c) on mesure l'activité isoenzymatique dans le précipité.

2. Procédé selon la revendication 1, caractérisé en ce que l'échantillon de liquide biologique est du sérum sanguin.

3. Procédé selon les revendications 1 et 2 caractérisé en ce que le premier anticorps est de l'anti-phosphatase prostatique acide de chèvre et en ce que le second anticorps est de l'IgG anti-chèvre d'âne.

4. Procédé selon l'une des revendications 1–3, caractérisé en ce qu'on détermine l'activité isoenzymatique par mesure spectrophotométrique.

5. Procédé selon la revendication 4, caractérisé en ce que la mesure spectrophotométrique comprend des réactifs colorimétriques.

6. Procédé selon la revendication 5, caractérisé en ce que les réactifs colorimétriques comprennent le monophosphate de thymolphtaléine ou un de ses sels d'addition basiques.

7. Système de trousse expérimentale diagnostique pour la détermination de la quantité de l'isoenzyme phosphatase prostatique acide dans un échantillon de liquide biologique, caractérisé en ce que le système de trousse expérimentale contient les composants suivants:

a) un premier récipient contenant un anticorps, qui est en situation de se lier sélectivement de façon immunologique sur l'isoenzyme,

b) un second récipient contenant un anticorps rendu insoluble formé par couplage de ce second anticorps sur l'amylose, où le second anticorps est en situation de réagir sélectivement de façon immunologique avec l'anticorps obtenu dans le premier récipient et

c) un réactif avec lequel on peut déterminer l'activité enzymatique de l'isoenzyme.

8. Procédé de préparation d'une composition faite de protéine et d'amylose, caractérisé en ce qu'on couple la protéine à l'amylose par l'intermédiaire d'un pont amine.

9. Procédé selon la revendication 8, caractérisé en ce que la protéine est une immunoglobuline.

**Claims for the Contracting States BE, CH, DE, FR, GB, IT, LI, NL, SE**

1. A process for determining the amount of prostatic acid phosphatase isoenzyme in a biological fluid sample, characterized by

a) incubating the sample with

(i) a soluble first antibody which selectively binds immunologically to the isoenzyme in the sample without inhibiting the enzymatic activity of this isoenzyme, and

(ii) an insolubilized antibody formed by coupling this second antibody to amylose whereby the second antibody selectively binds immunologically to the first antibody without inhibiting the enzymatic activity of the isoenzyme, to obtain a precipitate of a mixture of immunocomplexes which contain isoenzyme-first antibody-second antibody and first antibody-second antibody, and

b) isolating the precipitate containing the immunocomplexes and

c) measuring the isoenzyme activity in the precipitate.

2. A process according to claim 1, characterized in that the biological fluid sample is blood serum.

3. A process according to claim 1 or 2, characterized in that the first antibody is goat anti-prostatic acid phosphatase and the second antibody is donkey anti-goat IgG.

4. A process according to any one of claims 1–3, characterized in that the isoenzyme activity is measured by a spectrophotometric assay.

5. A process according to claim 4, characterized in that the spectrophotometric assay comprises colorimetric reagents.

6. A process according to claim 5, characterized in that the colorimetric reagents comprise thymolphthalein monophosphate or a basic addition salt thereof.

7. A diagnostic test kit system for determining the amount of prostatic acid phosphatase isoenzyme in a biological fluid sample, characterized in that the test kit system contains the following components:

a) a first container containing an antibody which is capable of selectively binding immunologically to the isoenzyme,

b) a second container containing an insolubilized antibody formed by coupling this second antibody to amylose, whereby the second antibody is capable of selectively reacting immunologically with the antibody contained in the first container, and

c) a reagent with which the enzymatic activity of the isoenzyme can be determined.

8. A composition consisting of a protein which is coupled to amylose via an amine bridge.

9. A composition according to claim 8, characterized in that the protein is an immunoglobulin.

10. A process for the manufacture of a composition according to claim 8 or 9, characterized by coupling the protein to the amylose via an amine bridge.

**Claims for the Contracting State AT**

1. A process for determining the amount of prostatic acid phosphatase isoenzyme in a biological fluid sample, characterized by

a) incubating the sample with

(i) a soluble first antibody which selectively binds immunologically to the isoenzyme in the sample without inhibiting the enzymatic activity of this isoenzyme, and

(ii) an insolubilized antibody formed by coupling this second antibody to amylose, whereby the second antibody selectively binds immunologically to the first antibody without inhibiting the enzymatic activity of the isoenzyme, to obtain a precipitate of a mixture of immunocomplexes which contain isoenzyme-first antibody-second antibody and first antibody-second antibody, and

b) isolating the precipitate containing the immunocomplexes and

c) measuring the isoenzyme activity in the precipitate.

2. A process according to claim 1, characterized in that the biological fluid sample is blood serum.

3. A process according to claim 1 or 2, characterized in that the first antibody is goat anti-prostatic acid phosphatase and the second antibody is donkey anti-goat IgG.

4. A process according to any one of claims 1–3, characterized in that the isoenzyme activity is measured by a spectrophotometric assay.

5. A process according to claim 4, characterized in that the spectrophotometric assay comprises colorimetric reagents.

6. A process according to claim 5, characterized in that the colorimetric reagents comprise thymolphthalein monophosphate or a basic addition salt thereof.

7. A diagnostic test kit system for determining the amount of prostatic acid phosphatase isoenzyme in a biological fluid sample, characterized in that the test kit system contains the following components:

a) a first container containing an antibody which is capable of selectively binding immunologically to the isoenzyme,

b) a second container containing an insolubilized antibody formed by coupling this second antibody to amylose, whereby the second antibody is capable of selectively reacting immunologically with the antibody contained in the first container, and

c) a reagent with which the enzymatic activity of the isoenzyme can be determined.

8. A process for the manufacture of a composition from a protein and amylose, characterized by coupling the protein to the amylose via an amine bridge.

9. A process according to claim 8, characterized in that the protein is an immunoglobulin.